# EUROPEAN PATENT APPLICATION

(11) **EP 1 352 950 A2**
(43) Date of publication of application: **15.10.2003**
(21) Application number: 03006911.6
(22) Date of filing: 26.03.2003
(51) Int. Cl.: C11D 17/04, A61K 7/48, A61K 7/50

(54) **Moist cleaning, skin care or cosmetic article**

(30) Priority: 11.04.2002 US 119837; 15.07.2002 US 194304
(71) Applicant: L'OREAL, 75008 Paris (FR)
(72) Inventor: Simon, Pascal, 94400 Vitry sur Seine (FR); Senee, Jérome, 91510 Lardy (FR)
(74) Representative: Tanty, François

(57) **Abstract**

An article which is particularly advantageous for use as a facial or personal care/cleansing article. The article is moist and includes a liquid composition impregnated in a substrate in an amount of 0.5 to 2.0 grams of the liquid composition per gram of substrate, preferably 1.0 to 1.5 grams of the liquid composition per gram of substrate. In a particularly preferred form, the liquid composition is 30 to 90% water, and further includes a surfactant and other ingredients. The article is particularly advantageous in that it can be utilized with or without the further addition of water, and moreover, the product is extremely consistent with little product variation due to storage as a result of settling of the liquid composition.

## Description

The invention relates to cleansing, care or cosmetic articles in which a liquid composition is retained by a substrate to provide a moist wipe article. The article is particularly advantageous as a personal care/cleansing article, however other uses are also possible.

Cleansing or personal care wipes have been known for many years. Such articles have been available as either wet wipes or dry wipes. Wet wipes are typically saturated or nearly saturated with a liquid composition, so that, when needed, the user can remove the article from a package and cleanse the skin with the article. Typically, wet wipes contain large amounts of a liquid composition, for example, with the liquid composition approximately three times the weight of the substrate. In addition, the substrate is typically a very thin substrate. However, with such a large amount of the liquid composition, particularly with a thin substrate, the substrate does not retain the liquid well and the liquid will settle from the substrate over time. As a result, the liquid composition is wasted as it settles from the substrate during storage. Moreover, the settled liquid can be inconvenient, for example, if the container should spill. In addition, if an antimicrobial or preservative substance is used, either an excessive amount of that substance must be provided to ensure sufficient antimicrobial/preservative properties despite settling, or alternatively, there is a risk that portions of the substrates from which the composition has settled will not be sufficiently protected. In addition, particularly where a group of wet wipes are packaged together, the product is inconsistent, because certain substrates can have more liquid than others.

Dry wipes have also been known for many years. For example, expired USP 4,303,543 to Mansy discloses impregnating a nonwoven cloth substrate with an aqueous solution of a surfactant and a conditioner, and the nonwoven cloth is subsequently dried to provide a dry wipe. When use of the article is desired, the user wets the substrate with water and proceeds to use the substrate to cleanse and condition the skin. Dry wipes can also be disadvantageous in a number of respects. In particular, dry wipes can be inconvenient if a water source is not readily available. In addition, if dry wipes are exposed to a moist environment (such as a steamy bathroom), the wipes can become tacky and stick together. Dry wipes are typically rigid before they are wetted, and therefore, can also be difficult to handle in manufacturing and packaging. Dry wipes can also be inconvenient in that they are packaged in an uncompressed state, thus requiring a large and cumbersome package which is inconvenient to carry, for example, in a purse or pocketbook. Moreover, attempting to package dry wipes in a compressed state can be problematic, because the rigidity of dry wipes is such that they cannot be easily maintained in a compressed state (they rapidly return to an uncompressed state after they are compressed).

The invention provides a wipe which is particularly advantageous as a personal care or personal cleansing article. It is to be understood, however, that certain aspects of the article could also be used, for example, for the application of a cosmetic, or for other uses as well, such as for household or industrial chores. The article is particularly advantageous as a wipe due to its consistency, ease of use, and ease of handling.

In a preferred example of the invention, the wipe article includes a substrate which preferably has at least 30% of hydrophilic fibers, more preferably at least 50% hydrophilic fibers. Although the substrate is preferably a nonwoven with at least 30% (more preferably at least 50%) hydrophilic fibers, the substrate could also be a woven cloth, a sponge, foam or other material. The substrate can be in the form of a sheet-like product, but could also be provided in various forms or shapes, such as in the form of a glove. The substrate can be a single layer or multilayer substrate, and can also include combinations of different types of substrates, for example, with combinations of sponge, cloth substrates, pervious substrates and impervious substrates.

Preferably, the wipe is impregnated with a liquid composition in the range of 50-200% by weight of the substrate or, in other words, 0.5-2.0 grams of liquid composition are provided per gram of substrate, and more preferably, 1.0-1.5 grams of the liquid composition are provided for each gram of substrate (100-150% impregnated). Preferably, the impregnated substrate is at least 20% by weight water. In addition, the liquid composition preferably includes 30-90% water, and most preferably 50-75% water.

The article is advantageous in that it is very consistent from substrate to substrate even when stacked and packaged as a group. Preferably, the wipe includes a surfactant, although wipe products with other ingredients are also contemplated. Where the wipe includes a surfactant, preferably the surfactant is a foaming surfactant. A nonlimiting list of foaming surfactants that can be used for the present invention is provided at the end of this specification. The article is also advantageous in that it can be advantageously utilized either with or without additional water. For example, if a water source is not available, the wipe can be utilized as a damp wipe due to the moisture content impregnated in the wipe. Water can also be added to the wipe, for example, if it is desired to form a substantial amount of foam. Thus, the arrangement of the invention achieves the benefits of both wet wipes and dry wipes, while avoiding the disadvantages.

In a further embodiment, the invention provides an article comprising:
a substrate having at least one layer formed of at least 30% hydrophilic fibers; and
a liquid composition impregnated in said substrate, wherein said liquid composition is 30 to 90% by weight water, and wherein said liquid composition further includes at least one surfactant.

In a further embodiment, the invention provides an article comprising:
a substrate having at least one layer;
a liquid composition impregnated into said at least one layer in an amount of 0.5 to 2.0 grams of said liquid composition per gram of said at least one layer; and
at least one hydrophilic active agent.

In a further embodiment, the invention provides an article comprising:
a substrate having at least one layer with at least 30% by weight of hydrophilic fibers;
a liquid composition impregnated in said at least one layer in an amount of 0.5 to 2.0 grams of said liquid composition per gram of said at least one layer, and wherein said liquid composition includes 30 to 90% by weight water, and further wherein said liquid composition includes at least one surfactant.

Figure 1 is a perspective view of an example of a wipe according to the invention; and
Figure 2 is a perspective view of an example of a package containing a plurality of wipes according to the invention.

In a preferred form of the invention, a liquid composition is held by a substrate to form a moist wipe article or product. Preferably, the article differs from conventional dry wipes in that it is neither dry nor substantially dry, and the article also differs from wet wipes in that it has a lower water or liquid content as compared with conventional wet wipe products.

Various substrates can be used according to the invention. In a presently preferred form, the substrate is a nonwoven substrate, preferably an apertured nonwoven substrate. However, other types of substrates could also be used, including nonwoven or woven cloth materials, foams, or sponges, and the substrates can include natural or synthetic materials or combinations of the foregoing. Preferably, the substrate (or at least one substrate layer) is formed of at least 30% hydrophilic fibers, more preferably at least 50% hydrophilic fibers.

The substrate can be a single layer substrate, or it can be formed of two or more layers. If, for example, the substrate is a multilayer substrate, the substrate could include both pervious and impervious layers, in which case at least one pervious layer is preferably formed of at least 30% hydrophilic fibers (more preferably at least 50%). As should be apparent, where the substrate is formed of multiple layers, the various layers can be the same or different. The substrates can also be of various forms or shapes, for example, with the substrate a rectangular or washcloth-like shape, or alternatively, the substrate can be in the form of a glove, mitt or mitten. Obviously, a wide variety of additional shapes are possible, such as oval, circular, etc.

By way of example, and not to be construed as limiting, examples of suitable substrates include Duralace 7006, Duralace 7123, Duralace 9796 (with Duralace available from PGI); Sontara 8021, Sontara 8801, Sontara 9951, Sontara 9957 (Sontara is available from DuPont Nonwovens); substrates available from Jacob Holm Ind. including: Norafin 1.73065.01, Lifast 55, ref. 321055 and LIDRO Bi-activ 70 g/m² (blanc), reference 1281-14; substrates available from Tharreau Ind. including Aquadim VE 50 G1 NL and Aquadim VE 75 G2 NL; and substrates produced by BBA including Ultraloft 182-010, Flexilon 140-130 and Novonette 149-807.

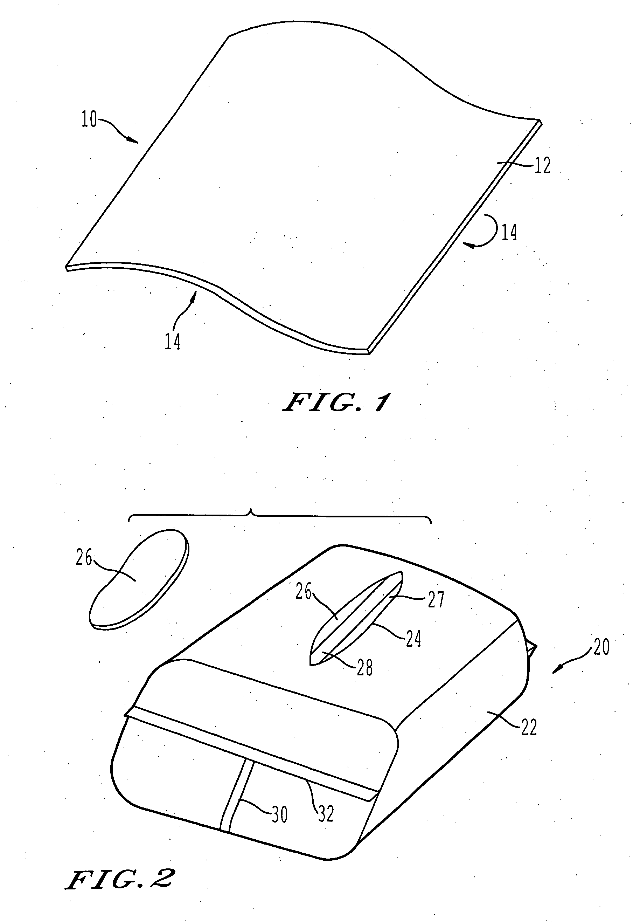

If desired, different surfaces of the substrate can have different surface roughnesses. For example, with reference to the example of an article 10 shown in Figure 1, it can be desirable to provide one surface 12 of the substrate with a smoother surface, while the opposite surface (the bottom surface, which is not seen in the figure) 14 is provided with a rougher surface. With this arrangement the user can select the surface most comfortable, or can use one surface for cleansing and another for exfoliating, etc. The provision of different surface roughnesses can be achieved in a multilayer substrate by forming the substrate with two substrate layers having different surface characteristics. Alternately, different surface roughnesses can be achieved in a single layer or multilayer substrate by performing a surface treatment step upon one or both sides of the substrate. The surface treatment can be, e.g., a mechanical treatment such as calendaring or embossing, or any other suitable surface treatment technique, such as by the deposition of a coating material, depositing fine balls or particles of polystyrene or other material, or by altering the surface roughness by a heat treatment operation. Thus, even where outer surfaces of the substrate are of the same material (either in a monolayer or in a multilayer substrate having outer layers which are the same), one surface of the wipe can be provided with different roughness/smoothness characteristics as compared with the opposite side of the substrate by a surface treatment or surface modification operation.

As noted earlier, in accordance with one of the advantageous aspects of the invention, it has been recognized that preferable performance is achieved if the substrate includes at least one layer having at least 30% hydrophilic fibers, and more preferably, at least 50% hydrophilic fibers. Where the substrate is a multilayer substrate, preferably each layer has at least 30%, more preferably at least 50%, hydrophilic fibers. However, for certain applications, the use of an impervious layer could be desired as a barrier layer, in which case the layers impregnated with the liquid preferably have the hydrophilic fibers. Substrates, with lower amounts of hydrophilic fibers have been found to be undesirable from an impregnation standpoint and from a standpoint of maintaining product consistency after impregnation. Also preferably, the substrate is apertured or porous.

As noted earlier, in accordance with one of the advantageous aspects of the invention, the substrate is impregnated with a liquid composition such that a moist product is provided which can be utilized either with or without the further addition of water. In addition, the substrate is impregnated with the liquid composition such that an extremely consistent product is provided, with the consistency maintained over a period of time. By contrast, conventional wet wipe products are susceptible to settling, which can result in inconsistencies from wipe to wipe, or even with certain portions of a given wipe inconsistent with respect to the remainder of the wipe. As used herein, "impregnated" or "impregnation" is used to mean that the liquid composition is held or retained by the substrate and is not limited to any particular mechanism by which the substrate holds the composition.

Preferably, the consistency of the product should be within 10%, in terms of the amount of change in weight of the substrate due to settling of the liquid over a period of time, such as during a two day test period. Thus, over a two day settling period, the settling variation (the weight change of the article due to settling) should not exceed 10%, preferably it should not exceed 5%. In accordance with an exemplary embodiment of the invention discussed in further detail hereinafter, an extremely consistent product has been achieved in which the settling variation was approximately 1% or less. By contrast, with conventional wet wipe products, settling variations have been in the range of 14-28%.

In terms of the amount of impregnation, preferably the liquid composition is impregnated into the substrate in an amount of 50%-200% in terms of the weight of the composition and the weight of the substrate. In other words, preferably 0.5-2.0 grams of liquid composition are provided for each gram of substrate. More preferably, the impregnation amount is 100% to 150% (1.0 to 1.5 grams of impregnated liquid composition impregnated per gram of substrate). In addition, preferably the composition includes substantial quantities of water. The water can be demineralized, sterilized, distilled, and/or filtered. In terms of the impregnated substrate (i.e., the substrate impregnated with the liquid composition), preferably the impregnated substrate is at least 20% by weight water. In terms of the liquid composition, preferably 30% to 90% by weight of the liquid composition is water, more preferably 40 to 80%, and most preferably 50-75%. These characteristics allow the product to be used either with or without adding additional water. In addition, these characteristics have been recognized as advantageous in providing a highly consistent product, even where the products are stacked upon one another, for example, for packaging and storage. An additional advantage of the arrangement of the invention is that the wipes can be packaged in a compressed stated. By contrast, conventional dry wipes are typically rigid such that if it is attempted to compress the wipes for packaging, they immediately spring back to their original form, making it difficult to package the wipes in a compressed state. Dry wipes are typically packaged in large cumbersome rigid plastic containers which are undesirable. With wipes according to the invention, they can be compressed, and after compression, they return to an uncompressed state slowly, so that the wipes can be conveniently compressed and packaged in a compressed state, with the package retaining the wipes in a compressed state. As a result, a smaller overall package size is achieved, making the product more convenient to carry, for example, in a purse, pocketbook, briefcase or suitcase.

Various forms of packages can be utilized. For example, a flexible plastic wrap package can be utilized to hold a plurality of articles in a compressed state, so that the articles can be conveniently carried. By way of example, Figure 2 depicts a suitable package 20 formed of a flexible wrap 22 and having an aperture 24 so that the wipes can be pulled one at a time through the aperture. Prior to the initial use, the aperture can be sealed, for example, with a film closure 26 that can have a tacky surface to hold the closure onto the package at least until the package is first opened or other suitable expedient (pull-cord opening, a puncture through or perforated opening, etc.). The closure can be replaceable, or discarded after the first use. Because the aperture 24 is relatively small, even where the topmost wipe is exposed to the aperture after the initial opening, the amount of potential drying is relatively small. In addition, particularly when initially opened, because the wipes are in a compressed state, the topmost wipe is urged against the aperture thereby further limiting amount of possible drying. If desired, the aperture in the package can be further limited by the provision of additional flexible plastic sheets 26, 27 extending along each adjacent edge of the aperture 24 to provide a flexible slit opening 28. Such flexible slit openings are known, for example, in the context of facial tissue boxes but have not been previously associated with wipe products, particularly wipe products in flexible packages. A plurality of wipes are preferably packaged in a package as shown, e.g., in Figure 2. The number of wipes can vary and, if desired, the wipes can be packaged individually. The wipes can be folded in various configurations. For example, the wipes can be interleaved with each other so that removing one wipe pulls a portion of the next wipe partially through the package aperture so that the package can be easily grasped.

The portions depicted at 30, 32 are seams resulting from forming such a package with form, fill and seal apparatus. It is to be understood that various package shapes, configurations, and manufacturing expedients are possible for a flexible wrap package and the package of Figure 2 is intended as an example. It is also to be understood that various forms of packaging are possible in addition to or in lieu of the flexible plastic packaging as described with reference to Figure 2. For example, the flexible plastic sheet packaging shown in Figure 2 can be provided within a paperboard or cardboard outer container. Alternately, the package of Figure 2 can be provided within a rigid plastic container, and optionally, with a paperboard or flexible wrap disposed around the rigid plastic container. As a further alternative, the wipes can be provided in a rigid container without a plastic wrap. As should be readily recognized, various packaging expedients are possible.

The wipe according to the invention can have numerous applications depending upon the ingredients associated with the wipe. For example, although the wipe is presently preferred for use as a facial or cleansing wipe, the wipe can be used for numerous additional or alternate purposes including, for example, make-up removal, application of make-up such as a foundation or other make-up products, sunscreen application or application of sunless tanning products, application of insect repellants, or for the application of other cosmetic or dermotologic compositions such as anti-acne, anti-aging/anti-wrinkle, first aid or anti-bacterial products. Thus, as should be readily understood, depending upon the ingredients associated with the wipe, the wipe can have numerous applications. The wipe could also be utilized for household or industrial chores, such as cleaning, polishing or dusting applications.

Where the wipe is to be utilized as a cleansing/facial wipe, liquid composition impregnated into the wipe preferably contains a foaming surfactant in an amount of greater than 10% by weight in terms of the weight of the liquid composition, preferably at least 15% and more preferably greater than 15%. Various types of surfactants are possible including ionic, anionic, cationic or amphoteric surfactants. The liquid composition impregnated into the wipe can also have various additional ingredients in combination with or in lieu of a surfactant such as one or more conditioners, vitamins, a cosmetic or make-up, a fragrance, neutralizing agents, various active agents (preferably hydrophilic active agents) and preservatives. As noted earlier, according to one of the advantages of the invention, the wipe can be used without requiring additional water. Where the liquid composition includes a surfactant, preferably sufficient water is provided in the liquid composition so that when the article is rubbed against a portion of the body a detergency action is initiated without requiring additional water (i.e., without requiring water added to that already present in the liquid composition impregnated into the substrate). If desired, supplemental water can also be added, for example, if a large amount of foaming is desired, or so that after cleansing the wipe can be used for rinsing. As used herein, a "detergency action" means an action that causes impurities such as pigments of a make-up composition, to be dispersed in the water, and/or oils or waxes forming such a make-up composition, to be emulsified with the water. According to another advantageous aspect of the invention, where supplemental water is available, initial cleansing can be performed by rubbing the article against the body, such as a face from which make-up (typically containing one or more of a pigment, an oil, and a wax) is to be removed. The water present in the impregnated article is sufficient to initiate a detergency action. After initial cleansing, water can be added to the substrate for further foaming of the substrate and cleansing of the skin upon rubbing against the face. By contrast, with dry articles, water must be added (e.g., under a faucet) for initial cleansing, and if further cleansing is desired, the second addition of water (e.g., under a faucet) can deplete the foam/surfactant such that the second or subsequent cleansing steps are unsatisfactory.

By way of example only, and not to be construed as limiting, an advantageous form of the invention was formed with a liquid composition containing an anionic surfactant in the form of sodium laureth sulfate (21%), a hydrophilic agent in the form of glycerine (5%), a fragrance (0.4%), a neutralizing agent in the form of triethanolamine (0.6%), an antioxidant in the form of disodium EDTA (0.1%), a hydrophilic active of menthol (0.3%), preservatives or antimicrobials in the form of methyl paraben (0.25%) and imidazolidinyl urea (0.25%), with additional hydrophilic actives of salicylic acid (0.6%) and laminaria saccharina extract (0.2%). The foregoing percentages are in terms of the percentage by weight of the liquid composition, and the remainder of the composition (71.3%) was water. It is to be understood that the foregoing ingredients and composition amounts are exemplary, and various compositions and the percentages of the ingredients can be used according to the invention.

The above composition was impregnated with an impregnation of 100% (or, in other words, 1 gram of liquid composition per gram of substrate) in a substrate known as Aquadim VE 75 G2 NL from Tharreau Industries, which is an apertured nonwoven substrate having weight of approximately 75 grams per square meter and a thickness of approximately 0.9 mm. Of course, as also discussed earlier, various other types of substrates can be used. The result was a wipe having a moist and fresh feel when handling, and the wipe was conveniently usable either with or without adding additional water (i.e., water in addition to the water already part of the liquid composition). If additional water is provided, substantial amounts of foam can be generated, with the wipe providing a fresh and more onctuous foam as compared with conventional wet wipes. Also, the wipe avoids a sticky feel as is the case with many conventional dry wipes. In addition, the article is advantageous from a standpoint of maintaining a consistent product during packaging and shipping.

To measure the consistency of the product after storage, 30 of the above wipes were stacked upon one another, and allowed to stand in an enclosure for a period of two days (the wipes were enclosed so that weight variations would be attributable to settling, as would be the case with a packaged product, rather than drying). Thereafter, the variation of the weight of the wipe as compared with the original weight of the wipe (with the weight of the wipe including both the weight of the substrate and the weight of the liquid composition) was measured. In weighing the wipes after settling, the top five wipes were weighed to provide an average of the weights of the top five wipes, and the bottom five wipes were also weighed to provide an average of the five bottommost wipes. As used herein, the term "settling variation" denotes a change in the average weight of the articles over a period of two days. As noted earlier, one of the undesirable aspects with conventional wet wipes is that, over time, the liquid settles from the wipe, so that much of the liquid remains in the bottom of the package, or alternatively, if the wipes are packaged as a stack, the uppermost wipes have a lower liquid content while the bottommost wipes have a higher liquid content. In accordance with the invention, it has been recognized that to provide desirable consistency characteristics, the settling variation should not exceed 10%, and preferably the settling variation should not exceed 5%. According to the example previously described, a consistent product was achieved, and the settling difference was surprisingly low, approximately 1% in terms of the change of the average weights of the topmost five and bottommost five wipes in the stack as compared with the original weight. By contrast, settling variations with wet wipes were observed as in the range of approximately 14 to 28% with wipes impregnated at a 300% impregnation level in non-apertured substrates having a weight of 50 grams per square meter. Thus, according to the invention, an advantageous and consistent product is achieved.

Other liquid compositions can be used in accordance with the present invention. By way of example, a first formulation in accordance with the invention includes: Water: 76.4%, Glycerol: 5%, Methyl paraben: 0.25%, Disodium EDTA: 0.1%, Sodium Laureth Sulfate (70% in water): 10%, Decyl Glucoside (53% in water): 8%, and Imidazolidinyl urea: 0.25%. As a further illustration, an example of a second formulation includes: Water: 62.8%, Glycerol: 5%, Methyl paraben: 0.25%, Disodium EDTA: 0.1%, Salicylic acid: 0.6%, Sodium Laureth Sulfate (70% in water): 30%, Imidazolidinyl urea: 0.25%, Fragrance: 0.4%, and Tri-Ethanolamine (99% in water): 0.6%. A third example includes: Water: 66.4%, Glycerol: 5%, Methyl paraben: 0.25%, Disodium EDTA: 0.1%, Salicylic acid: 0.6%, Sodium Laureth Sulfate (70% in water): 21%, Coco-Betaine (30% in water): 6%, Imidazolidinyl urea: 0.25%, Fragrance: 0.3%, and Menthol: 0.1%. The above formulations are provided as examples, and it is to be understood that other examples are possible, including formulations having additional ingredients and formulations in which one or more of the above ingredients are omitted.

For each of the examples above, the methyl paraben can be dissolved at 80EC in a water-glycerol mixture. The other ingredients can then be introduced successively after cooling to room temperature while stirring moderately. The substrate can then be 100% impregnated by the solution (1 gram of solution per gram of substrate). The substrate can be a nonwoven and can have a weight of, for example, 65 g/m². Also, by way of example, the substrate can be formed with hydro-entangled fibers, including viscose fibers (e.g., 65%) and polyester fibers (e.g., 35%).

A comparative test was performed on wipes according to the invention and conventional wipes. As an example of the invention, a substrate of Aquadim VE 75 G2 NL impregnated with the following solution: Water: 64%, Glycerol: 5%, Methyl paraben: 0.25%, Disodium EDTA: 0.1%, Sodium Laureth Sulfate (70% in water): 30%, Imidazolidinyl urea: 0.25%, and Fragrance: 0.4% was used to compare wipes according to the present invention with conventional dry wipes exposed to a relatively high humidity. Such an environment can be present, for example, in the moist environment of a steamy bathroom. Three different types of wipes were tested: (1) the wipes according to the present invention, (2) Oil of Olay™ dry wipes for normal-to-mixed skin with a bi-layer substrate, and (3) Oil of Olay™ dry wipes for dry skin with an apertured nonwoven substrate. Two stacks of wipes were tested for wipes (1) and (2). The wipes were examined after being exposed for 1 month at 37EC and 80% relative humidity (RH), then again after two months under the same conditions. For each type of wipe, a control set of wipes was placed under a glass enclosure at ambient conditions. The test results are illustrated in the following table:

| Type of wipe | Control After 2 months under ambient conditions | After 1 month at 37EC and 80% RH | After 2 months at 37EC and 80% RH |
|---|---|---|---|
| Stack # 1 of wipes according to the present invention-Open bag | Normal | Normal | Wipes on top of the stack are drier |
| Stack # 2 of wipes according to the present invention-Open bag | Normal | Normal | Wipes on top of the stack are drier |
| Stack #1 of Oil of Olay™ wipes for normal-to-mixed skin Open box | Normal | Slightly sticky | Slightly sticky |
| Stack #2 of Oil of Olay™ wipes for normal-to-mixed skin Open box | Slightly humid at the fold | Sticky and wet at the fold | Humid at the fold |
| Stack #1 of Oil of Olay™ wipes for dry skin - Open box | Slightly humid at the center | Wet and sticky at the fold | Slightly humid at the center |

As can be seen from these results, the wipes according to the present invention remained consistent or normal (no noticeable change in characteristics) after being exposed for one month at 37EC and 80% RH. The one month period is notable in that, for a consumer-size package with a stack of wipes that includes 30 wipes, using one wipe per day, the stack requires approximately one month to consume. Advantageously, all the wipes of such a stack of the invention would maintain their "normal" characteristics, even if exposed to a relatively high humidity for the entire period. In contrast, the conventional dry wipes become sticky after one month at 37EC and 80% RH.

After two months exposed at 37EC and 80% RH, only a relatively small number of wipes according to the present invention, which are located at the top of the stack, become drier. In contrast, the stack of conventional dry wipes are humid or sticky after two months at 37EC and 80% RH. The foregoing illustrates that the wipes according to the present invention maintain their consistent normal characteristics during a relatively long period of time, even after being exposed to a relatively high humidity. The present invention therefore provides an improved product over conventional wipes.

### Surfactants

For embodiments of the present invention that include a foaming composition, at least one surfactant is included. This surfactant may be chosen from, but need not be limited to, any nonionic, anionic, amphoteric or zwitterionic foaming surfactant and mixtures thereof.

The amount of surfactant(s) may range, for example, on an active material weight basis, from 2% to 50% by weight and better still from 3% to 30% by weight relative to the total weight of the composition.

### 1. Nonionic Surfactants:

Nonionic surfactants which may be used include, for example, alkyl polyglucosides (APGs), maltose esters, polyglycerolated fatty alcohols, glucamine derivatives such as 2-ethylhexyloxycarbonyl-N-methylglucamine, and mixtures thereof.

Alkyl polyglucosides which are preferably used are those containing an alkyl group containing from 6 to 30 carbon atoms and preferably from 8 to 16 carbon atoms, and containing a hydrophobic group (glucoside) preferably comprising from 1.2 to 3 saccharide units. Alkyl polyglucosides which could be used include, for example, decylglucoside (Alkyl-C9/C11-polyglucoside (1.4)), such as the product sold under the name Mydol 10® by the company Kao Chemicals, the product sold under the name Plantaren 2000 UP® by the company Henkel and the product sold under the name Oramix NS 10® by the company SEPPIC; caprylyl/capryl glucoside, such as the product sold under the name Oramix CG 110® by the company SEPPIC; laurylglucoside, such as the products sold under the names Plantaren 1200 N® and Plantacare 1200® by the company Henkel; and cocoglucoside, such as the product sold under the name Plantacare 818/UP® by the company Henkel.

The maltose derivatives can include, for example, those disclosed in document EP-A-566 438, such as O-octanoyl-6'-D-maltose or O-dodecanoyl-6'-D-maltose disclosed in document FR-2 739 556.

The polyglycerolated fatty alcohols can include, for example, polyglycerolated dodecanediol (3.5 mol of glycerol), this product being sold under the name Chimexane NF® by the company Chimex.

### 2. Anionic Surfactants:

Anionic surfactants may be chosen from carboxylates, amino acid derivatives, alkyl sulphates, alkyl ether sulphates, sulphonates, isethionates, taurates, sulphosuccinates, alkyl sulphoacetates, phosphates and alkyl phosphates, polypeptides, anionic alkyl polyglucoside derivatives and fatty acid soaps, and mixtures thereof.

Carboxylates can include, for example, alkali metal salts of N-acylamino acids; amido ether carboxylates (AECs), for instance sodium lauryl amido ether carboxylate (3 EO) sold under the name Akypo Foam 30® by the company Kao Chemicals; polyoxyethylenated carboxylic acid salts, for instance oxyethylenated (6 EO) sodium lauryl ether carboxylate (C12-14-16 65/25/10) sold under the name Akypo Soft 45 NV® by the company Kao Chemicals; polyoxyethylenated fatty acids of olive oil and of carboxymethyl, this product being sold under the name Olivem 400® by the company Biologia E Technologia; oxyethylenated (6 EO) sodium tridecyl ether carboxylate sold under the name Nikkol ECTD-6NEX® by the company Nikkol.

The amino acid derivatives may be chosen, for example, from sarcosinates and in particular acylsarcosinates, for instance the sodium lauroyl sarcosinate sold under the name Sarkosyl NL 97® by the company Ciba or sold under the name Oramix L 30® by the company SEPPIC, the sodium myristoyl sarcosinate sold under the name Nikkol Sarcosinate MN® by the company Nikkol or the sodium palmitoyl sarcosinate sold under the name Nikkol Sarcosinate PN® by the company Nikkol; alaninates, for instance the sodium N-lauroyl-N-methylamidopropionate sold under the name Sodium Nikkol Alaninate LN 30® by the company Nikkol or sold under the name Alanone ALE® by the company Kawaken, and the N-lauroyl-N-methylalanine triethanolamine sold under the name Alanone Alta® by the company Kawaken; N-acylglutamates, for instance the triethanolamine monococoylglutamate sold under the name Acylglutamate CT-12® by the company Ajinomoto and the triethanolamine lauroylglutamate sold under the name Acylglutamate LT-12® by the company Ajinomoto; aspartates, for instance the mixture of triethanolamine N-lauroyl aspartate and of triethanolamine N-myristoylaspartate, sold under the name Asparack® by the company Mitsubishi; citrates, and mixtures thereof.

Alkyl ether sulphates can include, for example, the sodium lauryl ether sulphate (C12-14 70/30) (2.2 EO) sold under the names Sipon AOS 225® or Texapon N702 PATE® by the company Henkel, the ammonium lauryl ether sulphate (C12-14 70/30) (3 EO) sold under the name Sipon Lea 370® by the company Henkel, and the ammonium (C12-C14) alkyl ether (9 EO) sulphate sold under the name Rhodapex AB/20® by the company Rhodia Chimie.

Sulphonates can include, for example, α-olefin sulphonates, for instance the sodium α-olefin sulphonate (C14-16) sold under the name Bio-Terge AS-40® by the company Stepan, sold under the names Witconate AOS Protege® and Sulframine AOS PH 12® by the company Witco or sold under the name Bio-Terge AS-40 CG® by the company Stepan, the sodium secondary olefin sulphonate sold under the name Hostapur SAS 30® by the company Clariant; linear alkyl aryl sulphonates, or the sodium xylenesulphonate sold under the names Manrosol SXS30®, Manrosol SXS40® and Manrosol SXS93® by the company Manro.

Isethionates can include, for example, acylisethionates, for instance sodium cocoylisethionate, such as the product sold under the name Jordapon CI P® by the company Jordan.

Taurates can include, for example, the sodium salt of palm kernel oil methyltaurate sold under the name Hostapon CT Pate® by the company Clariant; N-acyl N-methyltaurates, for instance the sodium N-cocoyl N-methyltaurate sold under the name Hostapon LT-SF® by the company Clariant or sold under the name Nikkol CMT-30-T® by the company Nikkol, and the sodium palmitoyl methyltaurate sold under the name Nikkol PMT® by the company Nikkol.

Sulphosuccinates can include, for example, the oxyethylenated (3 EO) lauryl monosulphosuccinate (C12/C14 70/30) sold under the names Setacin 103 Special®, Rewopol SB-FA 30 K 4® by the company Witco, the disodium salt of a C12-C14 alkyl hemisulphosuccinate, sold under the name Setacin F Special Paste® by the company Zschimmer Schwarz, the oxyethylenated (2 EO) disodium oleamidosulphosuccinate sold under the name Standapol HS 135® by the company Henkel, the oxyethylenated (5 EO) laurylamide monosulphosuccinate sold under the name Lebon A-5000® by the company Sanyo, the oxyethylenated (10 EO) disodium salt of lauryl citrate monosulphosuccinate sold under the name Rewopol SB CS 50® by the company Witco, and the ricinoleic monoethanolamide monosulphosuccinate sold under the name Rewoderm S 1333® by the company Witco.

Phosphates and alkyl phosphates can include, for example, monoalkyl phosphates and dialkyl phosphates, such as the lauryl monophosphate sold under the name MAP 20® by the company Kao Chemicals, the potassium salt of dodecylphosphoric acid, as a mixture of monoester and diester (mainly diester), sold under the name Crafol AP-31® by the company Cognis, the mixture of monoester and diester of octylphosphoric acid, sold under the name Crafol AP-20® by the company Cognis, the mixture of ethoxylated (7 mol of EO) phosphoric acid monoester and diester of 2-butyloctanol, sold under the name Isofol 12 7 EO-Phosphate Ester® by the company Condea, the potassium salt or triethanolamine salt of monoalkyl (C12-C13) phosphate sold under the references Arlatone MAP 230K-40® and Arlatone MAP 230T-60® by the company Uniqema, and the potassium lauryl phosphate sold under the name Dermalcare MAP XC-99/09® by the company Rhodia Chimie.

The polypeptides are obtained, for example, by coupling a fatty chain with amino acids from cereals and in particular from wheat and oat. Polypeptides can include, for example, the potassium salt of hydrolysed lauroyl wheat protein, sold under the name Aminofoam W OR® by the company Croda, the triethanolamine salt of hydrolysed cocoyl soybean protein, sold under the name May-Tein SY® by the company Maybrook, the sodium salt of oat lauroylamino acids, sold under the name Proteol Oat® by the company SEPPIC, the collagen hydrolysate grafted onto coconut fatty acid, sold under the name Geliderm 3000® by the company Deutsche Gelatine, and the soybean proteins acylated with hydrogenated coconut acids, sold under the name Proteol VS 22® by the company SEPPIC.

The anionic derivatives of alkylpoly-glucosides may be, in particular, glyceryl ethers, carbonates, sulphosuccinates, tartrates and citrates obtained from alkyl polyglucosides. For example, the sodium salt of cocoylpolyglucoside (1,4) tartaric ester, sold under the name Eucarol AGE-ET® by the company Cesalpinia, the disodium salt of cocoylpolyglucoside (1,4) sulphosuccinic ester, sold under the name Essai 512 MP® by the company SEPPIC, and the sodium salt of cocoylpolyglucoside (1,4) citric ester, sold under the name Eucarol AGE-EC® by the company Cesalpinia can be used.

The fatty acid soaps which may be used as anionic surfactants are fatty acids of natural or synthetic origin, salified with a mineral or organic base. The fatty chain may comprise from 6 to 22 carbon atoms and preferably from 8 to 18 carbon atoms. The mineral or organic base may be chosen from alkali metals or alkaline-earth metals, amino acids and amino alcohols. Salts which may be used, for example, include the sodium, potassium, magnesium, triethanolamine and N-methylglucamine salts of lysine and of arginine. Soaps which may be used, for example, include the potassium or sodium salts of lauric, myristic, palmitic or stearic acid (potassium or sodium laurate, myristate, palmitate and stearate), and mixtures thereof.

### 3. Amphoteric and Zwitterionic Surfactants:

Amphoteric and zwitterionic surfactants may be chosen, for example, from betaines, N-alkylamidobetaines and derivatives thereof, glycine derivatives, sultaines, alkyl polyaminocarboxylates and alkylamphoacetates, and mixtures thereof.

Betaines can include, for example, cocobetaine, for instance the product sold under the name Dehyton AB-30® by the company Henkel, laurylbetaine, for instance the product sold under the name Genagen KB® by the company Clariant, oxyethylenated (10 EO) laurylbetaine, for instance the product sold under the name Lauryl Ether (10 EO) Betaine® by the company Shin Nihon Rica, and oxyethylenated (10 EO) stearylbetaine, for instance the product sold under the name Stearyl Ether (10 EO) Betaine® by the company Shin Nihon Rica.

Among the N-alkylamidobetaines and derivatives thereof can include, for example, the cocamidopropylbetaine sold under the name Lebon 2000 HG® by the company Sanyo, or sold under the name Empigen BB® by the company Albright & Wilson, and the lauramidopropyl betaine sold under the name Rewoteric AMB12P® by the company Witco.

Glycine derivatives can include, for example, the sodium N-cocoylglycinate sold under the name Amilite GCS-12® by the company Ajinomoto.

Sultaines can include, for example, the cocoylamidopropylhydroxysulphobetaine sold under the name Crosultaine C-50 by the company Croda.

Alkyl polyaminocarboxylates (APACs) can include, for example, the sodium cocoylpolyaminocarboxylate sold under the name Ampholak 7 CX/C® and Ampholak 7 CX® by the company Akzo Nobel, the sodium stearylpolyamidocarboxylate sold under the name Ampholak 7 TX/C by the company Akzo Nobel and the sodium carboxymethyloleylpolypropylamine sold under the name Ampholak XO7/C® by the company Akzo Nobel.

Alkylamphoacetates can include, for example, N-disodium N-cocoyl-N-carboxymethoxyethyl-N-carboxymethylethylenediamine (CTFA name: disodium cocamphodiacetate), for instance the product sold under the name Miranol C2M Concentré NP® by the company Rhodia Chimie and N-sodium N-cocoyl-N-hydroxyethyl-N-carboxymethylethylenediamine (CTFA name: sodium cocamphoacetate).

### Active Agents

The active agents for the present invention may be chosen from the group comprising polyols such as glycerol; glycols, for instance butylene glycol, isoprene glycol, propylene glycol and polyethylene glycols such as PEG-8; sorbitol; sugars such as glucose, fructose, maltose, lactose or sucrose; and mixtures thereof; fragrances; preserving agents such as phenoxylethanol and parabens; sequestering agents (EDTA); pigments; nacres; mineral or organic fillers; matt-effect agents; bleaching or exfoliant agents; soluble colorants; sunscreens; vitamins such as retinol (vitamin A), ascorbic acid (vitamin C), tocopherol (vitamin E), niacinamide (vitamin B3, panthenol (vitamin B5) and tier derivatives; antiseptic; antiseborrhoeic agents such as oestrogens, cyproterone and its acetate, retinoids and aroretinoids (13-cis-retinoic acid), retinol and its derivatives, sulphur and sulphur-containing derivatives, benzoyl peroxide, zinc derivatives such as zinc sulphate, aluminium chloride, selenium disulphide and B vitamins, and mixtures thereof; antimicrobial agents such as benzoyl peroxide, salicylic acid, triclosan and azelaic acid; hydroxyacids such as citric acid and glycolic acid; optical brighteners; and mixtures thereof.

Obviously, numerous modifications and variations of the present invention are possible in light of the above teachings. It is therefore to be understood that, within the scope of the appended claims, the invention may be practiced otherwise than as specifically described herein.

## Claims

1. An article comprising:
a substrate;
a liquid composition impregnated into said substrate, wherein said impregnated substrate includes at least 20 % by weight of water; and
wherein said liquid composition is impregnated in said substrate in an amount in a range of 0.5 to 2.0 grams of said liquid composition per gram of substrate.

2. An article according to the preceding claim, wherein said substrate includes at least 30% hydrophilic fibers.

3. An article according to the preceding claim, wherein said substrate includes at least 50% by weight hydrophilic fibers.

4. An article according to any one of the preceding claims, wherein said substrate comprises a nonwoven substrate.

5. An article according to any one of claims 1 to 3, wherein said substrate comprises a woven substrate.

6. An article according to any one of the preceding claims, wherein said substrate comprises a sponge.

7. An article according to any one of the preceding claims, wherein said article is in the form of one of a glove and a mitt.

8. An article according to any one of the preceding claims, wherein said article has a settling variation not exceeding 10%, and wherein said settling variation is determined by stacking thirty of said substrates impregnated with said liquid composition upon one another to form a stack, allowing said stack to stand for two days, measuring a first average weight of a topmost five substrates and a second average weight of a bottommost five substrates in said stack, and wherein the first average weight and the second average weight vary 10% or less from an original weight of the substrates impregnated with said liquid composition.

9. An article according to the preceding claim, wherein said settling variation does not exceed 5%.

10. An article according to the preceding claim, wherein said settling variation does not exceed 1%.

11. An article according to any one of the preceding claims, wherein said liquid composition is present in said substrate in an amount of 1.0 to 1.5 grams of said composition per gram of substrate.

12. An article according to any one of the preceding claims, wherein said liquid composition is 30 to 90% by weight water.

13. An article according to the preceding claim, wherein said liquid composition includes 40 to 80% by weight water.

14. An article according to the preceding claim, wherein said liquid composition includes 50 to 75% by weight water.

15. An article according to any one of the preceding claims, further including a package within which said substrate is disposed, and wherein said substrate is at least partially compressed inside of said package.

16. An article according to the preceding claim, wherein a plurality of said substrates are disposed in said package in an at least partially compressed state.

17. An article according to any one of the two preceding claims, wherein said package is flexible.

18. An article according to any one of the preceding claims, wherein said liquid composition further includes at least one surfactant.

19. An article according to the preceding claim, wherein said liquid composition comprises at least 10% by weight of surfactant.

20. An article according to the preceding claim, wherein said surfactant is in an amount of at least 15% by weight of said liquid composition.

21. An article according to any one of the three preceding claims, wherein said at least one surfactant includes an anionic surfactant.

22. An article according to any one of the preceding claims, wherein said liquid composition includes laminaria saccharina extract.

23. An article according to any one of the preceding claims, wherein said liquid composition includes salicylic acid.

24. An article according to any one of the preceding claims, wherein said liquid composition includes at least one hydrophilic active agent.

25. An article according to the preceding claim, wherein said at least one hydrophilic active agent includes menthol.

26. An article according to any one of the two preceding claims, wherein said at least one hydrophilic active agent further includes salicylic acid.

27. An article according to any one of the preceding claims, wherein said liquid composition includes at least one antimicrobial.

28. An article according to any one of the preceding claims, wherein said liquid composition includes an antioxidant.

29. An article according to any one of the preceding claims, wherein said liquid composition includes:
(a) at least one foaming surfactant;
(b) at least one hydrophilic active agent; and
(c) at least one preservative.

30. An article according to any one of the preceding claims, wherein said liquid composition includes a cosmetic.

31. An article according to any one of the preceding claims, wherein said substrate is a multilayer substrate, and wherein at least one layer of said substrate contains at least 30% hydrophilic fibers.

32. An article according to the preceding claim, wherein at least one substrate layer includes at least 50% hydrophilic fibers.

33. An article according to any one of the preceding claims, wherein said substrate is a multilayer substrate, and wherein each layer of said substrate includes at least 30% hydrophilic fibers.

34. An article according to any one of the three preceding claims, wherein at least one layer is impregnated with said liquid composition in an amount in a range of 0.5 to 2.0 grams of said liquid composition per gram of said at least one layer.

35. An article according to any one of claims 31 to 34, wherein each substrate layer is impregnated with said liquid composition in an amount in a range of 0.5 to 2.0 grams of said liquid composition per gram of each respective substrate layer.

36. An article according to any one of claims 1 to 30, wherein said substrate is a single layer substrate.

37. An article comprising:
a substrate having at least one layer formed of at least 30% hydrophilic fibers; and
a liquid composition impregnated in said substrate, wherein said liquid composition is 30 to 90% by weight water, and wherein said liquid composition further includes at least one surfactant.

38. An article comprising:
a substrate having at least one layer;
a liquid composition impregnated into said at least one layer in an amount of 0.5 to 2.0 grams of said liquid composition per gram of said at least one layer; and
at least one hydrophilic active agent.

39. An article comprising:
a substrate having at least one layer with at least 30% by weight of hydrophilic fibers;
a liquid composition impregnated in said at least one layer in an amount of 0.5 to 2.0 grams of said liquid composition per gram of said at least one layer, and wherein said liquid composition includes 30 to 90% by weight water, and further wherein said liquid composition includes at least one surfactant.

40. A method of cleansing a body portion comprising the steps of:
a) providing an impregnated substrate comprising:
i) a substrate;
ii) a liquid composition including at least one surfactant, and
wherein said liquid composition is impregnated into said substrate, wherein the impregnated substrate includes at least 20 % by weight of water; and wherein said liquid composition is impregnated in said substrate in an amount in a range of 0.5 to 2.0 grams of said liquid composition per gram of substrate; and
b) rubbing said article against said body portion, wherein prior to or upon rubbing said article against said body portion, the article is contacted with additional water in order to cause a foaming of at least part of said at least one surfactant.

41. A method of cleansing a body portion comprising the steps of:
a) providing an impregnated substrate comprising:
i) a substrate;
ii) a liquid composition impregnated into said substrate, said liquid composition comprising at least one foaming surfactant and water in a sufficient amount to initiate a detergency action of said at least one surfactant when the impregnated substrate is rubbed against said body portion ;
b) rubbing said article against said body portion in order to at least partially clean said body portion;
c) contacting said rubbed article with water in order to cause a foaming of said at least one surfactant; and
d) rubbing said watered substrate against said body portion in order to have a further cleansing action of said body portion.

42. A method according to the preceding claim, wherein said body portion to be cleaned is covered, at least in part with at least one make-up product comprising pigments and/or an oil, and/or at least one wax.
